# EUROPEAN PATENT SPECIFICATION

(11) **EP 4 623 986 B1**
(45) Date of publication and mention of the grant of the patent: **05.08.2026**
(21) Application number: 24752698.1
(22) Date of filing: 24.01.2024
(51) Int. Cl.: A61N 1/36, A61N 1/04, A61N 1/32, A61F 13/08, A61H 9/00

(54) **LEG MASSAGE PATCH, PRESSURE STOCKING LEGS AND PRESSURE STOCKINGS**
BEINMASSAGEPFLASTER, DRUCKSTRUMPFBEIN UND DRUCKSTRUMPF
PATCH DE MASSAGE DE JAMBE, BAS DE CONTENTION POUR JAMBES ET BAS DE CONTENTION

(30) Priority: 09.02.2023 CN 202320206581 U
(43) Date of publication of application: 01.10.2025
(73) Proprietor: Jiaxing Max Hosiery Co., Ltd., Jiaxing, Zhejiang 314500 (CN)
(72) Inventor: ZHANG, Yuliang, Jiaxing, Zhejiang 314500 (CN)
(74) Representative: Chung, Hoi Kan
(86) International application number: PCT/CN2024/073754
(87) International publication number: WO 2024/164841

(56) References cited:
- WO-A1-2018/206067
- CN-A- 106 213 605
- CN-A- 109 464 273
- CN-A- 110 180 082
- CN-A- 110 251 833
- CN-U- 218 391 437
- CN-U- 219 375 884
- KR-A- 20100 049 382
- KR-A- 20210 048 453
- TW-U- M 583 297

## Description

### TECHNICAL FIELD

The present application relates to the field of leg massage techniques, and in particular, to a leg massage patch, a tube of compression stocking, and a compression stocking.

### BACKGROUND

Running and skipping have always been popular sports for most people, which mainly exercise the leg muscles. During running, the anterior muscles of the thigh, namely the quadriceps femoris muscle, including the vastus medialis muscle, the vastus lateralis muscle, and the vastus intermedius muscle and rectus femoris muscle between the vastus medialis muscle and the vastus lateralis muscle, will be frequently used. During skipping, the posterior muscles of the shank, the gastrocnemius muscle, will be frequently used. Therefore, it is necessary to perform appropriate massage for relaxation after the corresponding exercise process, to relieve the muscle soreness and other sports sequelae that will occur after the exercise.

One of the existing muscle massage methods is micro-current massage, which places a certain number of conductive patches on the corresponding leg surface, and then the control portion sends out low-frequency current pulses to simulate the current signals sent by the brain, to achieve the periodic contraction and relaxation of the corresponding muscles. For example, the patent with the publication No. CN2558224Y with the title of massage health underpants.

Regarding the above related technologies, the shape of the conductive patch is generally round or square, but the quadriceps femoris muscle or the gastrocnemius muscle that needs to be massaged for relaxation is not round or square, making it difficult for the conductive patch to fully cover the corresponding muscles during a micro-current massage, resulting in insufficient muscle massage.

WO 2018/206067 A1 discloses a compression garment, preferably a compression stocking, for providing neuromuscular electrical stimulation (NMES), the compression garment comprises at least two integrated electrode pads enabling NMES, wherein the integrated electrode pads comprise silicone polymers the silicone polymer further comprising graphene, the electrode pads being adapted to be in contact with the skin when in use. TW M 583 297 U discloses massage health underpants that feature conductive patches and massage pads for micro-current stimulation of specific body parts or acupoints. While primarily focused on areas like the waist, buttocks, and abdomen, this document generally teaches the concept and application of micro-current massage for muscle stimulation.

### SUMMARY

To massage muscles more sufficiently, the present application provides a leg massage patch, a tube of compression stocking, and a compression stocking.

According to a first aspect, the leg massage patch provided by the present application adopts the following technical solution.

A leg massage patch, including a conductive patch capable of performing a micro-current massage on muscles, where the conductive patch includes a thigh patch close to a quadriceps femoris muscle of a thigh of a user and a shank patch close to a gastrocnemius muscle of a shank of the user, two ends of the thigh patch in a length direction are close to, in a one-to-one correspondence manner, two ends of a vastus medialis muscle or a vastus lateralis muscle in a length direction, an edge of the thigh patch is of an arc shape to be close to an edge contour of the vastus medialis muscle or the vastus lateralis muscle, two ends of the shank patch in a length direction are close to, in a one-to-one correspondence manner, two ends of the gastrocnemius muscle in a length direction, and an edge of the shank patch is of an arc shape to be close to an edge contour of the gastrocnemius muscle.

By adopting the above technical solution, the thigh patch can fit the vastus medialis muscle or the vastus lateralis muscle comprehensively in view of a shape of the vastus medialis muscle or the vastus lateralis muscle, so that when two thigh patches are used to massage the thigh, the entire quadriceps femoris muscle can be massaged sufficiently. In addition, the shank patch can fit the gastrocnemius muscle comprehensively in view of a shape of the gastrocnemius muscle, so that the gastrocnemius muscle can be massaged comprehensively, to enable the muscles to be massaged more sufficiently.

Optionally, a side edge of the thigh patch in the length direction is of an arc shape, the arc-shaped side edge of the thigh patch is close to any one of two side edges that are away from each other of the vastus medialis muscle and the vastus lateralis muscle, and edges of the two ends of the thigh patch in the length direction are of arc shapes.

Optionally, the shank patch includes an upper portion region close to the thigh of the user and a lower portion region close to a foot of the user, a width of the upper portion region is greater than a width of the lower portion region, and edges around the upper portion region and the lower portion region are of arc shapes.

By adopting the above technical solution, the thigh patch and the shank patch are specifically designed according to shapes of corresponding muscles in human legs, so that areas of the thigh patch and the shank patch are not easy to have excessive unnecessary parts while the thigh patch can cover the corresponding muscles comprehensively, thereby reducing material wastes.

Optionally, the conductive patch further includes a support patch close to a tibialis anterior muscle of the shank, and the shank patch and the support patch release a current in sequence.

Optionally, the support patch is first operated for 0.1 s to 0.2 s, and then the shank patch is operated for 0.3 s to 0.4 s, to form a minimum action step; and an interval between every two action steps is 0.4 s to 0.6 s, and a cycle continues.

By adopting the above technical solution, sequential contraction of muscle groups on the shank during human walking is simulated, to relax the legs of the user after exercise according to an actual walking frequency.

Optionally, one shank patch and one support patch are disposed on each leg of the user correspondingly, one pushing patch and one support patch that are on a same leg of the user form a group of walking patches, and two groups of walking patches are operated in sequence.

By adopting the above technical solution, two shanks of the user can be contracted through current stimulation according to a contraction rule during actual human walking, thereby better simulating shank muscle massage of relaxing the legs and removing acid during human walking after exercise.

According to a second aspect, a tube of compression stocking provided by the present application adopts the following technical solution.

A tube of compression stocking, provided with the above leg massage patch, including: an upper tube body that is sleeved on and tightly attached to a thigh of a user, a lower tube body that is sleeved on and tightly attached to a shank of the user, a contact portion that is electrically connected to a thigh patch and a shank patch, and a control portion that is electrically connected to the contact portion and sends a periodic micro-current pulse to the contact portion, where two shank patches and two thigh patches are disposed, and the contact portion of the shank patch is further connected to a support patch.

By adopting the above technical solution, the upper tube body applies pressure to the thigh of the user and simultaneously cooperates with the thigh patch to massage thigh muscles of the user, so that the thigh of the user can obtain a better massage effect. The lower tube body has the same effect.

Optionally, a distance between upper portion regions of the two shank patches is greater than a distance between two lower portion regions.

By adopting the above technical solution, the two shank patches of a same lower tube body can better adapt to distributions of a medial head and a lateral head of the gastrocnemius muscle of one shank of a same user.

Optionally, both the upper tube body and the lower tube body are provided with the contact portion and the control portion, and the contact portion of the lower tube body is close to one shank patch.

By adopting the above technical solution, the upper tube body and the lower tube body can be worn and used separately, and a position of the contact portion of the lower tube body is designed to facilitate connecting the control portion to the contact portion on the lower tube body correspondingly by the user.

Optionally, the contact portion is fixedly connected to the upper tube body, and an elasticity modulus of the contact portion is greater than an elasticity modulus of the upper tube body.

By adopting the above technical solution, a connection between the contact portion and the conductive patch is more stable and less prone to damage, so that when the upper tube body is stretched, the contact portion is not prone to synchronous large stretching deformation. Therefore, in any state of the upper tube body, the control portion can be well connected to a preset position on the contact portion, so that a connection between the contact portion and the control portion is more stable.

Optionally, an air permeable region is formed on one side, away from a conductive patch, of the upper tube body, an elasticity modulus of the air permeable region is less than the elasticity modulus of the upper tube body, one air permeable region is disposed at each of two ends of the upper tube body correspondingly, and adjacent side edges of two air permeable regions are of arc shapes.

By adopting the above technical solution, a position at which the air permeable region is located is close to a back portion of the thigh of the user, so that when the upper tube body tightly encloses the thigh of the user, deformation of the air permeable region is large. Therefore, the overall air permeable performance is good, and the thigh of the user is not easy to be excessively stuffy. When the middle of the upper tube body maintains good pressure on the thigh of the user, the air permeable region also enables the thigh of the user to be not easy to be excessively stuffy, and the width of the air permeable region close to the middle position of the upper tube body is smaller, which is conducive to a stepped slow change in the pressure between the two ends and the middle of the upper tube body on the leg of the user and is not easy for the user to feel a large change in the pressure between the two ends and the middle when wearing the upper tube body, thereby improving comfort brought to the user when wearing the upper tube body.

According to a third aspect, a compression stocking provided by the present application adopts the following technical solution.

A compression stocking, provided with the above tube of compression stocking, where one end, close to a foot of a user, of a lower tube body is provided with a stocking body for the user to wear on the foot.

By adopting the above technical solution, the lower tube body and a stocking body are used jointly, to adapt to requirements of corresponding groups of people, the position of the upper tube body on the thigh is not prone to large deviation, to wear the upper tube body in place, and the upper tube body and the lower tube body can be used simultaneously or separately according to the requirements.

In conclusion, the present application has at least one beneficial effect as follows:

1. The thigh patch can fit the vastus medialis muscle or the vastus lateralis muscle comprehensively in view of a shape of the vastus medialis muscle or the vastus lateralis muscle, so that when two thigh patches are used to massage the thigh, the entire quadriceps femoris muscle can be massaged sufficiently. In addition, the shank patch can fit the gastrocnemius muscle comprehensively in view of a shape of the gastrocnemius muscle, so that the gastrocnemius muscle can be massaged comprehensively, to enable the muscles to be massaged more sufficiently.

2. The upper tube body applies pressure to the thigh of the user and simultaneously is matched with the thigh patch to massage thigh muscles of the user, so that the thigh of the user can obtain a better massage effect. The lower tube body has the same effect.

### BRIEF DESCRIPTION OF THE DRAWINGS

FIG. 1 is a schematic structural diagram of a thigh patch of a leg massage patch according to the present application;
FIG. 2 is a schematic structural diagram of a shank patch of a leg massage patch according to the present application;
FIG. 3 is a schematic structural diagram of a support patch of a leg massage patch according to the present application;
FIG. 4 is a schematic structural diagram of an inner wall side of an upper tube body in a tube of compression stocking according to the present application;
FIG. 5 is a schematic structural diagram of an inner wall side of a lower tube body in a tube of compression stocking according to the present application;
FIG. 6 is a schematic structural diagram of another side of a lower tube body in FIG. 5 to show a support patch;
FIG. 7 is a schematic structural diagram of an inner wall side of an upper tube body which is different from that in FIG. 4 in a tube of compression stocking according to the present application; and
FIG. 8 is a schematic structural diagram of a compression stocking according to the present application.

Reference numerals: 1. conductive patch; 2. contact portion; 21. support patch; 23. walking patch; 3. control portion; 31. air permeable region; 32. lower tube body; 33. stocking body; 34. upper tube body; 35. thigh patch; 36. shank patch; 37. lower portion region; 38. upper portion region.

### DETAILED DESCRIPTION OF THE EMBODIMENTS

The present invention is defined by the appended claims. Further aspects, embodiments and examples described hereinafter are only of exemplary nature and are presented for better understanding the present invention. The present application is further described in detail below with reference to the accompanying drawings.

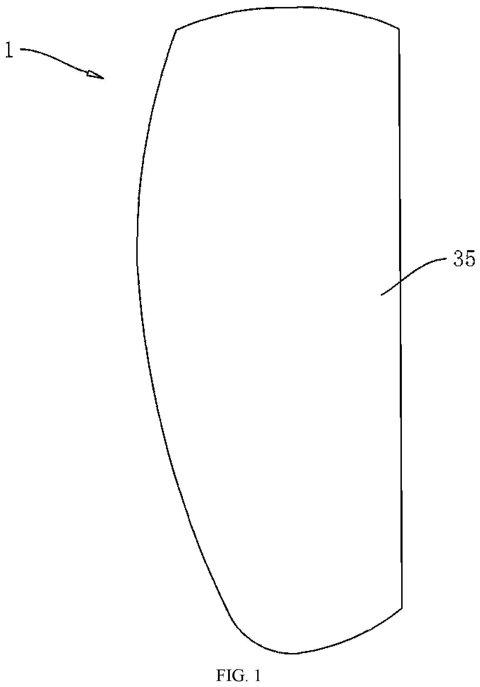

An embodiment of the present application discloses a leg massage patch. Referring to FIG. 1 to FIG. 2, the leg massage patch includes a conductive patch 1 that fits a surface of a leg of a user, and the conductive patch 1 may be an electrotherapy cloth that can generate a certain deformation. The conductive patch 1 includes a thigh patch 35 and a shank patch 36, the thigh patch 35 fits a front portion of a thigh of the user, and the shank patch 36 fits a rear portion of a shank of the user, to perform a micro-current massage on corresponding muscles of the thigh and the shank of the user.

Referring to FIG. 1, two ends of the thigh patch 35 in a length direction are close to, in a one-to-one correspondence manner, two ends of a vastus medialis muscle or the vastus lateralis muscle in a length direction. A side edge of one end of the thigh patch 35 in a width direction is of an arc shape, and one end of the arc-shaped side edge of the thigh patch 35 in the width direction is close to any arch-shaped side edge of two side edges that are away from each other of the vastus medialis muscle and the vastus lateralis muscle. A straight edge of one end of the thigh patch 35 in the width direction is close to any arc-shaped side edge of two side edges that are close to each other of the vastus medialis muscle and the vastus lateralis muscle.

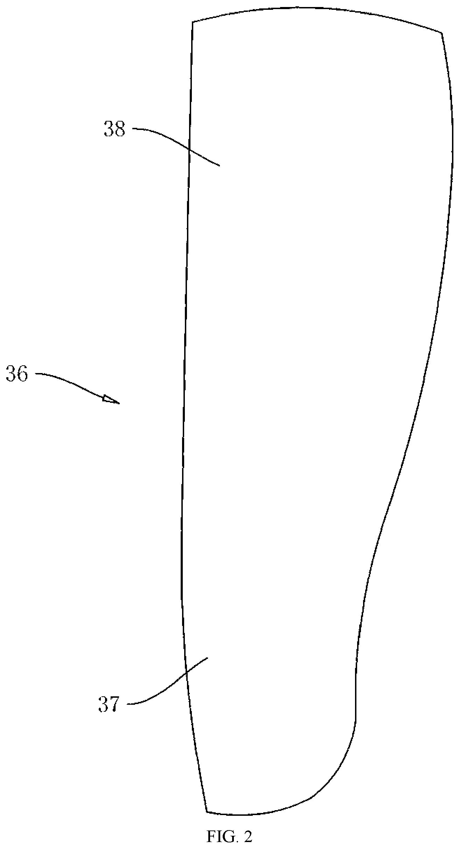

Referring to FIG. 2, the shank patch 36 includes an upper portion region 38 close to the thigh of the user and a lower portion region 37 close to a foot of the user, and the upper portion region 38 and the lower portion region 37 are close to, in a one-to-one correspondence manner, two ends of a gastrocnemius muscle in a length direction. The width of the upper portion region 38 is greater than the width of the lower portion region 37, edges of the upper portion region 38 and the lower portion region 37 are of arc shapes, and a joint between the upper portion region 38 and the lower portion region 37 is of an arc shape for transition.

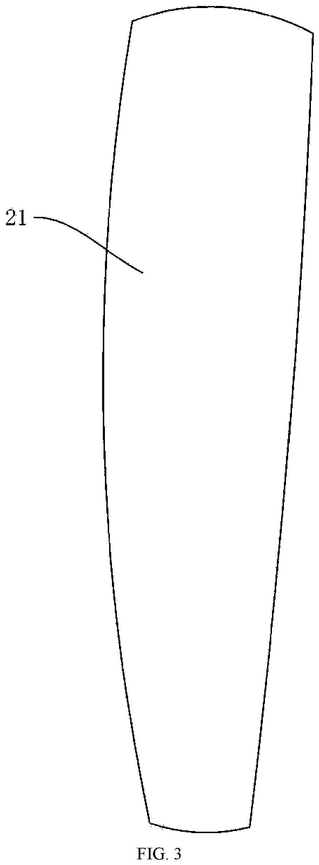

Referring to FIG. 3, the conductive patch 1 further includes a support patch 21 close to a tibialis anterior muscle of the shank. The shank patch 36 and the support patch 21 release a current in sequence. The support patch 21 is first operated for 0.1 s to 0.2 s, and then the shank patch 36 is operated for 0.3 s to 0.4 s, to form a minimum action step; and an interval between every two minimum action steps is 0.4 s to 0.6 s, and a cycle continues. In addition, one shank patch 36 and one support patch 21 are disposed on each leg of the user correspondingly, one shank patch 36 and one support patch 21 that are on the same leg of the user form a group of walking patches 23, and two groups of walking patches 23 are operated in sequence.

For example, when simulating human walking, it is assumed that a left leg is moved first. When a left foot is in contact with the ground, the support patch 21 of a left shank releases a current at this time to stimulate the tibialis anterior muscle to contract for 0.2 s, to play a role of stabilizing the body. Then a right leg is moved, and the shank patch 36 of the left shank releases a current at this time to stimulate the gastrocnemius muscle to contract for 0.4 s, to play a role of pushing the human body to move forward. When the right leg is in contact with the ground, the support patch 21 of a right shank releases a current to stimulate the tibialis anterior muscle to contract for 0.2 s, to play a role of stabilizing the body. Then the left leg is moved, and a cycle continues, to simulate muscle contraction during human walking.

An implementation principle of the leg massage patch of the embodiment of the present application is as follows: two thigh patches 35 may be used to fit the vastus medialis muscle and the vastus lateralis muscle, so that the vastus medialis muscle, the vastus lateralis muscle, and a vastus intermedius muscle and a rectus femoris muscle that are between the vastus medialis muscle and the vastus lateralis muscle can be comprehensively subjected to the micro-current massage effect of two conductive patches 1. The shank patch 36 can adaptively and comprehensively cover a medial head and a lateral head of the gastrocnemius muscle of the user.

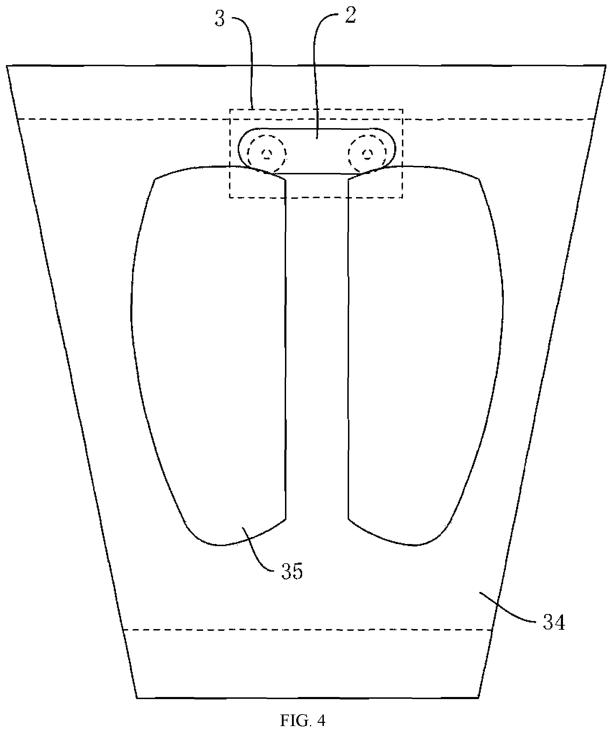

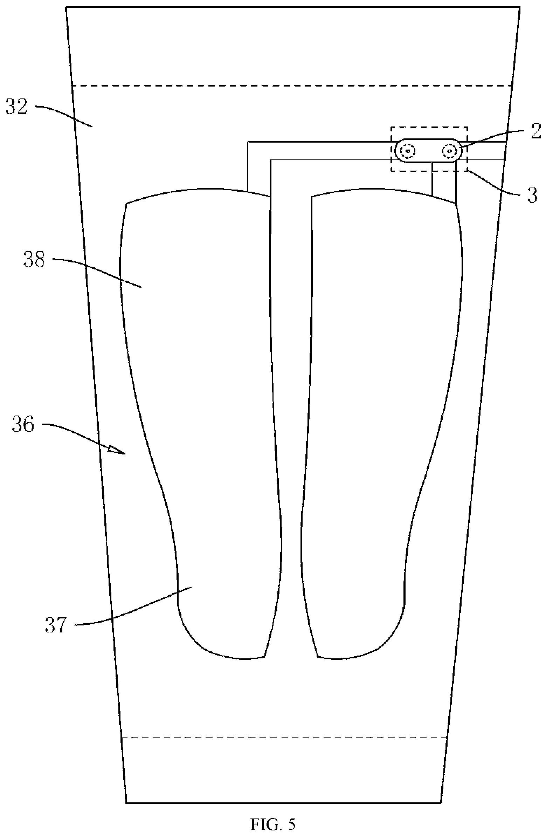

An embodiment of the present application further discloses a tube of compression stocking, provided with the above leg massage patch. Referring to FIG. 4 and FIG. 5, the tube of compression stocking includes an upper tube body 34 that is tightly sleeved on a thigh of a user and a lower tube body 32 that is tightly sleeved on a shank of the user. According to requirements, the upper tube body 34 and the lower tube body 32 can be integrally formed or separated to be used separately. Two thigh patches 35 are attached to an inner wall of the upper tube body 34, and two shank patches 36 are attached to an inner wall of the lower tube body 32. Each of the upper tube body 34 and the lower tube body 32 is provided with a contact portion 2, the contact portion 2 may be an insulating cloth and two metal electrode sheets, and the contact portion 2 of the upper tube body 34 is electrically connected to the two corresponding thigh patches 35.

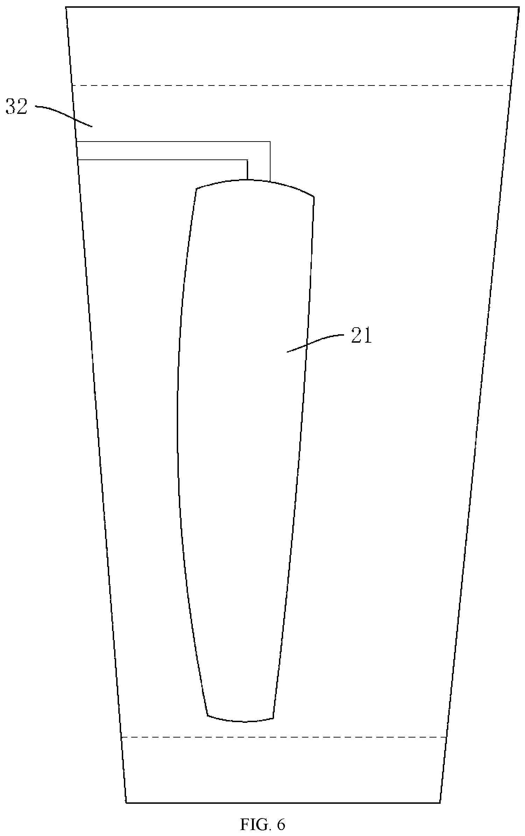

Referring to FIG. 5 and FIG. 6, the contact portion 2 of the lower tube body 32 is electrically connected to the two corresponding shank patches 36 and one support patch 21, and the metal electrode sheet of the contact portion 2 is exposed outside the upper tube body 34 or the lower tube body 32. The contact portion 2 may be detachably connected to a control portion in a magnetic attraction manner, and the control portion 3 may be a pulse generator with a built-in power supply.

Referring to FIG. 4 and FIG. 5, the two thigh patches 35 cover the vastus medialis muscle and the vastus lateralis muscle in a one-to-one correspondence manner, and adjacent straight edges of the two thigh patches 35 in the length direction are close to, in a one-to-one correspondence manner, two sides of the vastus intermedius muscle and the rectus femoris muscle in a length direction. A distance between upper portion regions 38 of the two shank patches 36 is greater than a distance between two lower portion regions 37. The contact portion 2 of the lower tube body 32 is close to the upper portion region 38 of one shank patch 36, and the contact portion 2 is away from the lower portion region 37. At the same time, an elasticity modulus of the contact portion 2 is far greater than an elasticity modulus of the upper tube body 34, so that the contact portion 2 is not prone to deformation, and a distance between the two metal electrode sheets of the contact portion 2 is not prone to change, thereby facilitating connecting the control portion 3 to the two metal electrode sheets of the contact portion 2.

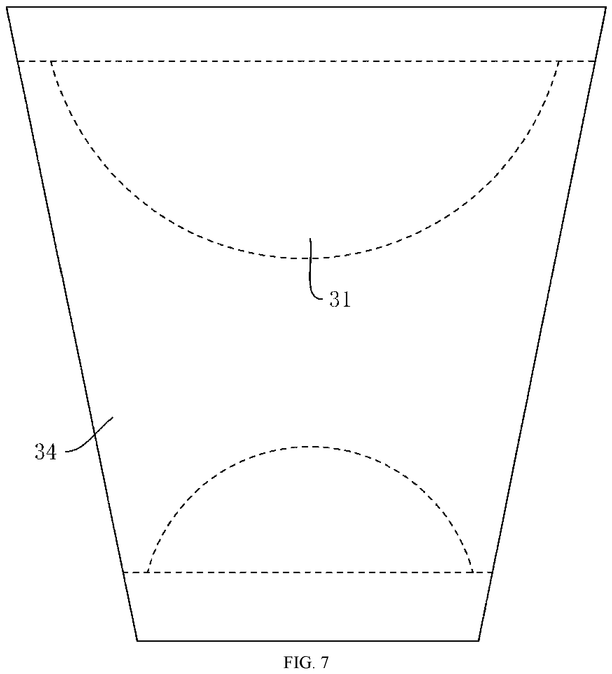

Referring to FIG. 7, two air permeable regions 31 are formed at positions, away from the thigh patch 35, of the upper tube body 34, and the two air permeable regions 31 are located at two ends of the upper tube body 34 in a one-to-one correspondence manner. Knitting quality of the air permeable region 31 is less than knitting quality of the upper tube body 34, so that an elasticity modulus of the air permeable region 31 is less than the elasticity modulus of the upper tube body 34, air permeable performance at the air permeable region 31 is better, and the two ends of the upper tube body 34 are looser. Adjacent side edges of the two air permeable regions 31 are of arch shapes, so that pressure on the leg of the user gradually increases from the two ends of the upper tube body 34 to the middle.

An implementation principle of the tube of compression stocking of the embodiment of the present application is as follows: the upper tube body 34 is matched with the thigh patch 35, and the lower tube body 32 is matched with the shank patch 36, to better massage and relax the leg of the user.

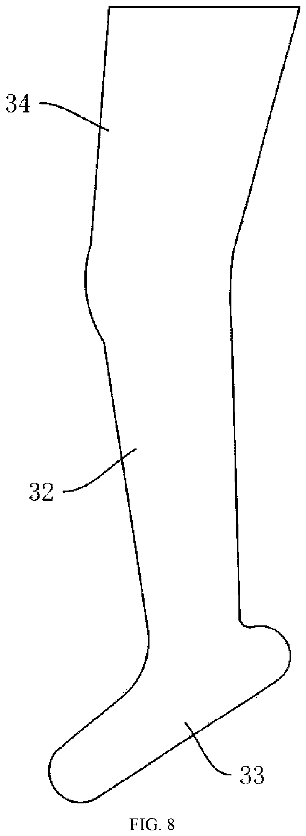

An embodiment of the present application further discloses a compression stocking, provided with the above tube of compression stocking. Referring to FIG. 8, one end, close to a foot of a user, of a lower tube body 32 is provided with a stocking body 33 in an integrally-formed manner. The stocking body 33 fits the foot of the user for the user to wear on the foot. One end, away from the stocking body 33, of the lower tube body 32 may be integrally formed on an upper tube body 34 according to requirements, and the upper tube body 34 may be separated from the lower tube body 32.

An implementation principle of the compression stocking of the embodiment of the present application is as follows: the compression stocking can adapt to requirements of different users and helps a wearing position of the lower tube body 32 or the upper tube body 34 not to have large deviation.

## Claims

1. A leg massage patch, comprising a conductive patch (1) capable of performing a micro-current massage on muscles, **characterized in that** the conductive patch (1) comprises a thigh patch (35) close to a quadriceps femoris muscle of a thigh of a user and a shank patch (36) close to a gastrocnemius muscle of a shank of the user, two ends of the thigh patch (35) in a length direction are close to, in a one-to-one correspondence manner, two ends of a vastus medialis muscle or a vastus lateralis muscle in a length direction, an edge of the thigh patch (35) is of an arc shape to be close to an edge contour of the vastus medialis muscle or the vastus lateralis muscle, two ends of the shank patch (36) in a length direction are close to, in a one-to-one correspondence manner, two ends of the gastrocnemius muscle in a length direction, and an edge of the shank patch (36) is of an arc shape to be close to an edge contour of the gastrocnemius muscle;
wherein, a side edge of the thigh patch (35) in the length direction is of an arc shape, the arc-shaped side edge of the thigh patch (35) is close to any one of two side edges that are away from each other of the vastus medialis muscle and the vastus lateralis muscle, and edges of the two ends of the thigh patch (35) in the length direction are of arc shapes.

2. The leg massage patch according to claim 1, **characterized in that** the shank patch (36) comprises an upper portion region (38) close to the thigh of the user and a lower portion region (37) close to a foot of the user, a width of the upper portion region (38) is greater than a width of the lower portion region (37), and edges around the upper portion region (38) and the lower portion region (37) are of arc shapes.

3. The leg massage patch according to claim 1, **characterized in that** the conductive patch (1) further comprises a support patch (21) close to a tibialis anterior muscle of the shank, and the shank patch (36) and the support patch (21) release a current in sequence.

4. The leg massage patch according to claim 3, **characterized in that** the support patch (21) is first operated for 0.1 s to 0.2 s, and then the shank patch (36) is operated for 0.3 s to 0.4 s, to form a minimum action step; and an interval between every two action steps is 0.4 s to 0.6 s, and a cycle continues.

5. The leg massage patch according to claim 4, **characterized in that** one shank patch (36) and one support patch (21) are disposed on each leg of the user correspondingly, one pushing patch and one support patch (21) that are on a same leg of the user form a group of walking patches (23), and two groups of walking patches (23) are operated in sequence.

6. A tube of compression stocking, suitable for being provided with the leg massage patch according to claim 1, **characterized by** comprising:
an upper tube body (34) that is sleeved on and tightly attached to a thigh of a user, a lower tube body (32) that is sleeved on and tightly attached to a shank of the user, a contact portion (2) that is electrically connected to a thigh patch (35) and a shank patch (36), and a control portion (3) that is electrically connected to the contact portion (2) and sends a periodic micro-current pulse to the contact portion (2), wherein two shank patches (36) and two thigh patches (35) are disposed, and the contact portion (2) of the shank patch (36) is further connected to a support patch (21).

7. The tube of compression stocking according to claim 6, **characterized in that** a distance between upper portion regions (38) of the two shank patches (36) is greater than a distance between two lower portion regions (37).

8. The tube of compression stocking according to claim 6, **characterized in that** both the upper tube body (34) and the lower tube body (32) are provided with the contact portion (2) and the control portion (3), and the contact portion (2) of the lower tube body (32) is close to one shank patch (36).

9. The tube of compression stocking according to claim 6, **characterized in that** the contact portion (2) is fixedly connected to the upper tube body (34), and an elasticity modulus of the contact portion (2) is greater than an elasticity modulus of the upper tube body (34).

10. The tube of compression stocking according to claim 6, **characterized in that** an air permeable region (31) is formed on one side, away from a conductive patch (1), of the upper tube body (34), an elasticity modulus of the air permeable region (31) is less than the elasticity modulus of the upper tube body (34), one air permeable region (31) is disposed at each of two ends of the upper tube body (34) correspondingly, and adjacent side edges of two air permeable regions (31) are of arc shapes.

11. A compression stocking system, comprising a compression stocking and the tube of compression stocking according to claim 6, **characterized in that** the compression stocking comprises a stocking body (33) for a user to wear on a foot, and the stocking body (33) is integrally formed with an end of the lower tube body (32) of the tube of compression stocking.

## Patentansprüche

1. Beinmassage-Elektrodenpad, umfassend ein leitfähiges Elektrodenpad (1), das in der Lage ist, eine Mikrostrommassage an Muskeln durchzuführen, **dadurch gekennzeichnet, dass** das leitfähige Elektrodenpad (1) ein Oberschenkel-Elektrodenpad (35) nahe einem Musculus quadriceps femoris eines Oberschenkels eines Benutzers und ein Unterschenkel-Elektrodenpad (36) nahe einem Musculus gastrocnemius eines Unterschenkels des Benutzers umfasst, wobei zwei Enden des Oberschenkel-Elektrodenpads (35) in Längsrichtung in Eins-zu-eins-Entsprechung nahe zwei Enden eines Musculus vastus medialis oder eines Musculus vastus lateralis in einer Längsrichtung liegen, eine Kante des Oberschenkel-Elektrodenpads (35) eine Bogenform aufweist, um nahe einer Kantenkontur des Musculus vastus medialis oder des Musculus vastus lateralis zu liegen, zwei Enden des Unterschenkel-Elektrodenpads (36) in einer Längsrichtung in Eins-zu-eins-Entsprechung nahe zwei Enden des Musculus gastrocnemius in einer Längsrichtung liegen und eine Kante des Unterschenkel-Elektrodenpads (36) eine Bogenform aufweist, um nahe einer Kantenkontur des Musculus gastrocnemius zu liegen;
wobei eine Seitenkante des Oberschenkel-Elektrodenpads (35) in Längsrichtung eine Bogenform aufweist, die bogenförmige Seitenkante des Oberschenkel-Elektrodenpads (35) nahe einer beliebigen von zwei voneinander abgewandten Seitenkanten des Musculus vastus medialis und des Musculus vastus lateralis liegt und Kanten der zwei Enden des Oberschenkel-Elektrodenpads (35) in Längsrichtung Bogenformen aufweisen.

2. Beinmassage-Elektrodenpad nach Anspruch 1, **dadurch gekennzeichnet, dass** das Unterschenkel-Elektrodenpad (36) einen oberen Abschnittsbereich (38) nahe dem Oberschenkel des Benutzers und einen unteren Abschnittsbereich (37) nahe einem Fuß des Benutzers umfasst, eine Breite des oberen Abschnittsbereichs (38) größer ist als eine Breite des unteren Abschnittsbereichs (37) und Kanten um den oberen Abschnittsbereich (38) und den unteren Abschnittsbereich (37) Bogenformen aufweisen.

3. Beinmassage-Elektrodenpad nach Anspruch 1, **dadurch gekennzeichnet, dass** das leitfähige Elektrodenpad (1) ferner ein Stütz-Elektrodenpad (21) nahe einem Musculus tibialis anterior des Unterschenkels umfasst und das Unterschenkel-Elektrodenpad (36) und das Stütz-Elektrodenpad (21) nacheinander einen Strom abgeben.

4. Beinmassage-Elektrodenpad nach Anspruch 3, **dadurch gekennzeichnet, dass** das Stütz-Elektrodenpad (21) zuerst für 0,1 s bis 0,2 s betrieben wird und anschließend das Unterschenkel-Elektrodenpad (36) für 0,3 s bis 0,4 s betrieben wird, um einen minimalen Aktionsschritt zu bilden; und ein Intervall zwischen jeweils zwei Aktionsschritten 0,4 s bis 0,6 s beträgt und ein Zyklus fortgesetzt wird.

5. Beinmassage-Elektrodenpad nach Anspruch 4, **dadurch gekennzeichnet, dass** jeweils ein Unterschenkel-Elektrodenpad (36) und ein Stütz-Elektrodenpad (21) entsprechend an jedem Bein des Benutzers angeordnet sind, ein Schub-Elektrodenpad und ein Stütz-Elektrodenpad (21), die an demselben Bein des Benutzers angeordnet sind, eine Gruppe von Geh-Elektrodenpads (23) bilden und zwei Gruppen von Geh-Elektrodenpads (23) nacheinander betrieben werden.

6. Kompressionsstrumpfschlauch, der dazu geeignet ist, mit dem Beinmassage-Elektrodenpad nach Anspruch 1 versehen zu werden, **dadurch gekennzeichnet, dass** dieser umfasst:
einen oberen Schlauchkörper (34), der auf einen Oberschenkel eines Benutzers aufgezogen und eng daran angebracht ist, einen unteren Schlauchkörper (32), der auf einen Unterschenkel des Benutzers aufgezogen und eng daran angebracht ist, einen Kontaktabschnitt (2), der elektrisch mit einem Oberschenkel-Elektrodenpad (35) und einem Unterschenkel-Elektrodenpad (36) verbunden ist, und einen Steuerabschnitt (3), der elektrisch mit dem Kontaktabschnitt (2) verbunden ist und einen periodischen Mikrostromimpuls an den Kontaktabschnitt (2) sendet, wobei zwei Unterschenkel-Elektrodenpads (36) und zwei Oberschenkel-Elektrodenpads (35) angeordnet sind und der Kontaktabschnitt (2) des Unterschenkel-Elektrodenpads (36) ferner mit einem Stütz-Elektrodenpad (21) verbunden ist.

7. Kompressionsstrumpfschlauch nach Anspruch 6, **dadurch gekennzeichnet, dass** ein Abstand zwischen oberen Abschnittsbereichen (38) der zwei Unterschenkel-Elektrodenpads (36) größer ist als ein Abstand zwischen zwei unteren Abschnittsbereichen (37).

8. Kompressionsstrumpfschlauch nach Anspruch 6, **dadurch gekennzeichnet, dass** sowohl der obere Schlauchkörper (34) als auch der untere Schlauchkörper (32) mit dem Kontaktabschnitt (2) und dem Steuerabschnitt (3) versehen sind und der Kontaktabschnitt (2) des unteren Schlauchkörpers (32) nahe einem Unterschenkel-Elektrodenpad (36) liegt.

9. Kompressionsstrumpfschlauch nach Anspruch 6, **dadurch gekennzeichnet, dass** der Kontaktabschnitt (2) fest mit dem oberen Schlauchkörper (34) verbunden ist und ein Elastizitätsmodul des Kontaktabschnitts (2) größer ist als ein Elastizitätsmodul des oberen Schlauchkörpers (34).

10. Kompressionsstrumpfschlauch nach Anspruch 6, **dadurch gekennzeichnet, dass** ein luftdurchlässiger Bereich (31) an einer von einem leitfähigen Elektrodenpad (1) abgewandten Seite des oberen Schlauchkörpers (34) ausgebildet ist, ein Elastizitätsmodul des luftdurchlässigen Bereichs (31) kleiner ist als das Elastizitätsmodul des oberen Schlauchkörpers (34), jeweils ein luftdurchlässiger Bereich (31) entsprechend an jedem von zwei Enden des oberen Schlauchkörpers (34) angeordnet ist und benachbarte Seitenkanten von zwei luftdurchlässigen Bereichen (31) Bogenformen aufweisen.

11. Kompressionsstrumpfsystem, umfassend einen Kompressionsstrumpf und den Kompressionsstrumpfschlauch nach Anspruch 6, **dadurch gekennzeichnet, dass** der Kompressionsstrumpf einen Strumpfkörper (33) umfasst, den ein Benutzer an einem Fuß tragen kann, und der Strumpfkörper (33) einstückig mit einem Ende des unteren Schlauchkörpers (32) des Kompressionsstrumpfschlauchs ausgebildet ist.

## Revendications

1. Patch de massage de jambe, comprenant un patch conducteur (1) capable d'effectuer un massage par micro-courants sur les muscles, **caractérisé en ce que** le patch conducteur (1) comprend un patch de cuisse (35) proche d'un muscle quadriceps fémoral de la cuisse d'un utilisateur et un patch de tibia (36) proche d'un muscle gastrocnémien d'un tibia de l'utilisateur ; deux extrémités du patch de cuisse (35), dans le sens de la longueur, sont proches, en correspondance biunivoque, de deux extrémités d'un muscle vaste médial ou d'un muscle vaste latéral, dans le sens de la longueur, un bord du patch de cuisse (35) est en forme d'arc de façon à approcher un contour de bord du muscle vaste médial ou du muscle vaste latéral, deux extrémités du patch de tibia (36), dans le sens de la longueur, sont proches, en correspondance biunivoque, de deux extrémités du muscle gastrocnémien, dans le sens de la longueur, et un bord du patch de tibia (36) est en forme d'arc de façon à approcher le contour de bord du muscle gastrocnémien ;
dans lequel, un bord latéral du patch de cuisse (35), dans le sens de la longueur, est en forme d'arc, le bord latéral en forme d'arc du patch de cuisse (35) est proche de l'un quelconque des deux bords latéraux qui sont éloignés l'un de l'autre du muscle vaste médial et du muscle vaste latéral, et des bords des deux extrémités du patch de cuisse (35), dans le sens de la longueur, sont en forme d'arc.

2. Patch de massage de jambe selon la revendication 1, **caractérisé en ce que** le patch de tibia (36) comprend une région de partie supérieure (38) près de la cuisse de l'utilisateur et une région de partie inférieure (37) près du pied de l'utilisateur, une largeur de la région de partie supérieure (38) est plus grande qu'une largeur de la région de partie inférieure (37), et les bords autour de la région de partie supérieure (38) et de la région de partie inférieure (37) sont en forme d'arc.

3. Patch de massage de jambe selon la revendication 1, **caractérisé en ce que** le patch conducteur (1) comprend en outre un patch de support (21) près d'un muscle tibial antérieur du tibia, et le patch de tibia (36) et le patch de support (21) libèrent un courant en séquence.

4. Patch de massage de jambe selon la revendication 3, **caractérisé en ce que** le patch de support (21) est d'abord activé pendant 0,1 s à 0,2 s, puis le patch de tibia (36) est activé pendant 0,3 s à 0,4 s, pour former une étape d'action minimale ; et un intervalle entre chaque deux étapes d'action est de 0,4 s à 0,6 s, et un cycle se poursuit.

5. Patch de massage de jambe selon la revendication 4, **caractérisé en ce qu'**un patch de tibia (36) et un patch de support (21) sont disposés sur chaque jambe de l'utilisateur de manière correspondante, un patch de poussée et un patch de support (21) qui se trouvent sur une même jambe de l'utilisateur forment un groupe de patchs de marche (23), et deux groupes de patchs de marche (23) sont activés en séquence.

6. Tube de bas de contention, adapté pour être pourvu d'un patch de massage de jambe selon la revendication 1, **caractérisé en ce qu'**il comprend :
un corps de tube supérieur (34) qui est enfilé sur et attaché fermement à une cuisse d'un utilisateur, un corps de tube inférieur (32) qui est enfilé sur et attaché fermement à une jambe de l'utilisateur, une partie de contact (2) qui est reliée électriquement à un patch de cuisse (35) et à un patch de tibia (36), et une partie de commande (3) qui est reliée électriquement à la partie de contact (2) et envoie une impulsion de micro-courant périodique à la partie de contact (2), dans lequel deux patchs de tibia (36) et deux patchs de cuisse (35) sont disposés, et la partie de contact (2) du patch de tibia (36) est en outre reliée à un patch de support (21).

7. Tube de bas de contention selon la revendication 6, **caractérisé en ce qu'**une distance entre des régions de partie supérieure (38) des deux patchs de tibia (36) est supérieure à une distance entre deux régions de partie inférieure (37).

8. Tube de bas de contention selon la revendication 6, **caractérisé en ce que** le corps de tube supérieur (34) et le corps de tube inférieur (32) sont tous les deux pourvus de la partie de contact (2) et de la partie de commande (3), et la partie de contact (2) du corps de tube inférieur (32) est proche d'un patch de tibia (36).

9. Tube de bas de contention selon la revendication 6, **caractérisé en ce que** la partie de contact (2) est reliée de manière fixe au corps de tube supérieur (34), et un module d'élasticité de la partie de contact (2) est supérieur à un module d'élasticité du corps de tube supérieur (34).

10. Tube de bas de contention selon la revendication 6, **caractérisé en ce qu'**une région perméable à l'air (31) est formée sur un côté, à l'écart d'un patch conducteur (1), du corps de tube supérieur (34), un module d'élasticité de la région perméable à l'air (31) est inférieur au module d'élasticité du corps de tube supérieur (34), une région perméable à l'air (31) est disposée au niveau de chacune des deux extrémités du corps de tube supérieur (34) de manière correspondante, et des bords latéraux adjacents des deux régions perméables à l'air (31) sont en forme d'arc.

11. Système de bas de contention, comprenant un bas de contention et le tube de bas de contention selon la revendication 6, **caractérisé en ce que** le bas de contention comprend un corps de bas (33) qu'un utilisateur porte sur un pied, et le corps de bas (33) est formé d'un seul tenant avec une extrémité du corps de tube inférieur (32) du tube de bas de contention.
